Europäisches Patentamt

European Patent Office

Office européen des brevets

(11) Publication number: **0 481 087 B1**

# EUROPEAN PATENT SPECIFICATION

(45) Date of publication of patent specification: **12.07.95**

(51) Int. Cl.⁶: **C08F 20/04**, A61K 49/00, G01R 33/28

(21) Application number: **91907701.6**

(22) Date of filing: **19.04.91**

(86) International application number: **PCT/JP91/00525**

(87) International publication number: **WO 91/16380 (31.10.91 91/25)**

(54) MOLDED BODY OF WATER-ABSORBING RESIN, PROCESS FOR PRODUCING MOLDED BODY, USE THEREOF, AND PROCESS FOR PREPARING WATER-ABSORBING RESIN.

(30) Priority: **20.04.90 JP 105950/90**
**30.07.90 JP 203640/90**
**10.08.90 JP 212939/90**
**28.09.90 JP 262902/90**

(43) Date of publication of application:
**22.04.92 Bulletin  92/17**

(45) Publication of the grant of the patent:
**12.07.95 Bulletin  95/28**

(84) Designated Contracting States:
**DE FR GB IT NL**

(56) References cited:
**DE-A- 3 614 142**
**JP-A- 1 260 014**
**JP-A- 6 032 830**
**JP-A-63 242 345**
**JP-B- 6 148 521**

(73) Proprietor: **ARAKAWA CHEMICAL INDUS-TRIES, LIMITED**
**21, Hirano-machi 1-chome**
**Higashi-ku**
**Osaka-shi**
**Osaka-fu (JP)**

(72) Inventor: **Tanioku, Shozo**
**128-1, Hagiwara, Haibaracho**
**Uda-gun, Nara 633-02 (JP)**
Inventor: **Oshima, Nobuyuki**
**1-8-21, Shiginonishi, Joto-ku**
**Osaka-Shi, Osaka 536 (JP)**
Inventor: **Takeuchi, Kenji**
**3-1-23, Moroguchi, Tsurumi-ku**
**Osaka-shi, Osaka 538 (JP)**
Inventor: **Maeda, Yoshiko**
**3-12-2, Nagata, Joto-ku**
**Osaka-shi, Osaka 536 (JP)**
Inventor: **Kitagawa, Akihiro**
**3-26-33, Hagioka**
**Hamamatsu-shi, Shizuoka 433 (JP)**

EP 0 481 087 B1

(74) Representative: **Barz, Peter, Dr. et al**
**Patentanwalt**
**Kaiserplatz 2**
**D-80803 München (DE)**

**Description**

The present invention relates to novel molded bodies of water-absorbing resin, process for producing the molded body, use thereof, and process for preparing the water-absorbing resin.

Water-absorbing resins are used for sanitary products, diapers, disposable dustcloth and like sanitary goods, as water retaining agents in agriculture or gardening, and for coagulation of sludge, prevention of dew condensation on building materials or dehydration of oils. Crosslinked polymers of acrylic acid alkali metal salts are primarily used as such water-absorbing resins.

On the other hand, molded bodies which swell with water are demanded in the field of toys and materials for simulated human bodies for medical purposes, such as phantoms for NMR diagnosis. The characteristics required of such molded bodies are, e.g., excellent water-absorbing capacity, excellent uniformity, high transparency, strength and freedom from air bubbles.

Water-absorbing resins are considered to be suitable as materials for these molded bodies, whereas water-absorbing resins generally known are powdery and have the drawback that when the resin is melted for molding, the molecular chains of the resin break to seriously impair the water-absorbing ability of the resin, which therefore fails to exhibit the contemplated properties as a water-absorbing resin. Although molded bodies of a blend of thermoplastic resin and water-absorbing resin are known, the molded body has very insufficient ability to swell with water and in no way fulfills the foregoing requirements. JP-A-21052/1988 mentions that a molded body of swollen powder of water-absorbing resin is usable as a phantom for NMR diagnosis. However, with the molded body prepared from merely swollen powder of water-absorbing resin, the molecules of the swollen water-absorbing resin are not interlocked with one another but permit formation of air bubbles or like air layer therebetween, and the molded body is not of uniform structure in its entirety. Accordingly, the molded body has the drawback of being low in strength and in transparency.

Presently, therefore, molded bodies still remain to be developed which fulfill the foregoing requirements as to characteristics.

The present applicant has already disclosed a process (JP-A-43912/1988) for producing a water-absorbing resin continuously and very efficiently, for example, on an endless belt from a mixture of aqueous monomer solution containing an acrylic acid alkali metal salt and a divinyl compound (crosslinking agent), and a photosensitizer (photoinitiator) which is a water-soluble azo compound having an amidino group, by irradiating the mixture with ultraviolet rays for polymerization and crosslinking. The process is almost free of the likelihood that the resin obtained will be porous or water solubles, such as low-molecular-weight substances, will be formed. The applicant made investigations to obtain molded bodies of water-absorbing resin which would fulfill the foregoing characteristics requirements by the application of this production process, and consequently found that the process permitted air bubbles to occur in the water-absorbing resin obtained in the form of a gel, and was not capable of satisfying all the requirements. It was also found that the formation of low-molecular-weight substances cannot always be prevented completely.

Such molded bodies which swell with water are useful, for example, as medical phantoms for NMR diagnosis. These phantoms are reference samples required for objectively evaluating and judging the maintenance and adjustment of NMR apparatus for use in diagnosing lesions and bloodstreams within the living body and reading images without errors. NMR images to be used for diagnosis are inferior in stability to conventional X-ray CT, so that care must always be taken of the maintenance, inspection, adjustment and evaluation of performance of the apparatus. Further since the adjustment and operation of the apparatus, and the method of forming images with the apparatus have not been standardized, difficulties are frequently encountered in analyzing images. In such a case, therefore, there arises a need to use NMR diagnosis phantoms.

Such phantoms need to satisfy the requirements of, for example, giving sharp NMR images, being similar to the living structure in NMR data (e.g. proton concentration (water content), spin-lattice (longitudinal) relaxation time (T1) and spin-spin (transverse) relaxation time (T2)), and being excellent in shape retentivity (more specifically, the strength of gel itself and stability thereof with time) as models of the living body, handleability at room temperature and moldability.

Up to date, polyvinyl alcohol water-containing gels, and rats or like living bodies are chiefly used as such phantoms. However, these phantoms have drawbacks; difficulties are encountered in suitably adjusting the water content, it is cumbersome or impossible to mold models of the living body, and they are poor in shape retentivity or handleability.

Although JP-A-21052/1988 mentions that a molded body of swollen powder of water-absorbing resin is usable as an NMR phantom as previously stated, the molecules of swollen resin are not interlocked with one another but permit presence of an air bubble or air layer therebetween to make the phantom

3

EP 0 481 087 B1

nonuniform in its entirety, making it difficult to obtain accurate NMR characteristics. Further the molded body has the drawbacks of being low in shape retentivity, handleability and moldability.

An object of the present invention is to provide a novel molded body of a water-absorbing resin, the body being excellent in water-absorbing capacity and uniformity, having high transparency and strength and being substantially free from air bubbles.

Another object of the invention is to provide a process for producing a novel molded body of a water-absorbing resin having such excellent characteristics.

Another object of the invention is to provide a phantom comprising such a molded body of water-absorbing resin for use in NMR diagnosis.

Another object of the invention is to provide a novel process for producing a water-absorbing resin substantially free from air bubbles and with a greatly reduced likelihood of forming water solubles as of low molecular weight.

These and other objects of the present invention will become apparent from the following description.

More specifically, the present invention provides :

(1) a molded body of a water-absorbing resin characterized in that the water-absorbing resin is a crosslinked polymer of acrylic acid or a crosslinked polymer of an acrylic acid alkali metal salt and forms a uniform matrix phase wherein the molecules of the resin are interlocked with one another, and water absorbed by the resin is uniformly dispersed as water chemically and/or physically bonded to the resin, and that the body is substantially free from air bubbles (as specified in more detail below);

(2) a process for producing the molded body set forth in the above paragraph (1) characterized by cooling, solidifying and molding a mixture of a photoinitiator and an aqueous monomer solution containing acrylic acid and/or an alkali metal salt of acrylic acid, and a crosslinking agent, and irradiating the molded mixture with ultraviolet rays to polymerize and crosslink the mixture;

(3) a process for producing the molded body set forth in the above paragraph (1) characterized by irradiating a mixture of a photoinitiator evolving no gas under polymerization and crosslinking conditions and an aqueous monomer solution containing acrylic acid and/or an alkali metal salt of acrylic acid, and a crosslinking agent with ultraviolet rays for polymerization and crosslinking to obtain a water-absorbing resin, and molding the resin into a predetermined shape, or by irradiating the mixture with ultraviolet rays for polymerization and crosslinking while holding the mixture in a predetermined shape,

(4) a phantom for NMR diagnosis characterized in that the phantom comprises a molded body set forth in the paragraph (1), and

(5) a process for producing a water-absorbing resin characterized by cooling and solidifying a mixture of a photoinitiator and an aqueous monomer solution containing acrylic acid and/or an alkali metal salt of acrylic acid, and a crosslinking agent, and irradiating the solidified mixture with ultraviolet rays to polymerize and crosslink the mixture.

In view of the situation of the foregoing prior art, we have further conducted intensive research to develop a novel molded body of water-absorbing resin which fully fulfills the requirements of being excellent in water-absorbing capacity and uniformity, having high transparency and strength and being substantially free from air bubbles, and obtained the following novel findings.

(1) When the mixture of aqueous monomer solution and photoinitiator for use in the production process of JP-A-43912/1988 is cooled, solidified and molded before irradiation with ultraviolet rays, a water-absorbing resin molded body of desired shape can be obtained easily which is substantially free from air bubbles and greatly reduced in the content of water solubles such as substances of low molecular weight.

(2) When the photoinitiator used in this case is one which does not evolve gas under polymerization and crosslinking conditions, a water-absorbing resin molded body of desired shape and free from air bubbles can be obtained easily without cooling and solidifying the mixture.

(3) Acrylic acid is usable in place of the alkali metal salt of acrylic acid.

(4) The molded body obtained is excellent in characteristics such as water-absorbing capacity, and also excellent in shape retentivity, moldability and handleability, has NMR characteristics equivalent to or same as those of the living body, exhibits a sharp NMR image and is well suited as a phantom for NMR diagnosis.

(5) When cooled, solidified and thereafter irradiated with ultraviolet rays, the mixture affords a water-absorbing resin which is substantially free from air bubbles and greatly reduced in the contents of low-molecular-weight substances or like water solubles.

The present invention has been accomplished based on these novel findings.

The molded body of water-absorbing resin of the present invention is in its entirety in the form of a uniform matrix phase wherein the molecules of the resin are interlocked with one another. This means that

4

the water-absorbing resin forming the molded body is formed by a single gel wherein molecular chains of the resin are present as interlocked with one another. The resin has water absorbed thereby and uniformly dispersed in the matrix phase, as water chemically and/or physically bonded with the resin. The term "chemically bonded" refers to a hydrogen bond between the water-absorbing resin and the dispersed water. The term "physically bonded" refers to so-called free water not associated, for example, with the hydrogen bond and taken into the water-absorbing resin in an energetically stable state.

The molded body of the water-absorbing resin is further substantially free from air bubbles. The expression "substantially free from air bubbles" means that no air bubbles are observable with the unaided eye. However, up to three air bubbles, not larger than 0.1 mm in diameter, may be present per $cm^3$ of the molded body within the permissible range of the invention.

In view of the foregoing, the molded body of the present invention comprises a water-absorbing resin and water which are uniformly present throughout the body, and is substantially free from air bubbles. Accordingly, the molded body of the invention is a novel molded body of a water-absorbing resin which is excellent in water-absorbing capacity and uniformity and is satisfactory in transparency, has strength and is free from air bubbles, thus fully fulfilling the desired requirements.

The water-absorbing resin for the molded body of the invention is a crosslinked product of an acrylic acid polymer or a crosslinked product of a polymer of an alkali metal salt of acrylic acid. The molded body can be produced suitably, for example, by the foregoing process (2) or (3) of the invention.

Said production processes (2) and (3) will be described first.

The main monomer unit for forming the water-absorbing resin of the present invention is at least one of acrylic acid and an alkali metal salt of acrylic acid. According to the present invention, acrylic acid can be used as unneutralized or as neutralized completely to 100%. The alkali metal salt is e.g. the sodium salt or potassium salt and is obtained by neutralizing acrylic acid with e.g. sodium hydroxide or potassium hydroxide. In view of the water-absorbing capacity of the water-absorbing resin, the degree of neutralization is in the range of 50 to 100%, preferably in the range of 60 to 85%. On the other hand, unneutralized acrylic acid polymer has water-absorbing ability and is usable as a water-absorbing resin free of trouble.

The crosslinking agent for use in the invention, when copolymerized and/or crosslinked with the monomer unit, gives a crosslinked structure to the water-absorbing resin obtained. Examples of such crosslinking agents are divinyl compounds, polyepoxy compounds and polyaziridinyl compounds. These agents are used singly, or at least two of them are used in combination.

Examples of useful divinyl compounds are divinylbenzene, N,N'-methylenebisacrylamide, N,N'-methylenebismethacrylamide, polyethylene glycol diacrylate and polypropylene glycol diacrylate.

Examples of polyepoxy compounds are diglycidyl ethers and triglycidyl ethers. Examples of diglycidyl ethers are (poly)ethylene glycol diglycidyl ether, (poly)propylene glycol diglycidyl ether, 1,6-hexanediol diglycidyl ether, glycerin-1,3-diglycidyl ether and neopentyl glycol diglycidyl ether. Preferable among these are, for example, (poly)ethylene glycol diglycidyl ether and (poly)propylene glycol diglycidyl ether. Preferably the (poly)alkylene glycol diglycidyl ethers are 2 to 50 in the polymerization degree of alkylene oxide. Examples of triglycidyl ethers are trimethylolpropane triglycidyl ether and glycerin triglycidyl ether. Such compounds having a larger number of functional groups are, e.g., polyglycerin polyglycidyl ether, sorbitol polyglycidyl ether and pentaerythritol polyglycidyl ether. Preferable among these are trimethylolpropane triglycidyl ether, glycerin triglycidyl ether and polyglycerin polyglycidyl ether. Examples of useful polyepoxy compounds further include epichlorohydrin-modified products of polyamide-polyamine condensates.

Examples of polyaziridinyl compounds are those having at least two aziridinyl groups, such as tetramethylolmethane-tri-$\beta$-aziridinyl propionate, trimethylolpropane-tri-$\beta$-aziridinyl propionate and 4,4'-bis-(ethyleneimino-carbonylamino)diphenylmethane.

The amount of crosslinking agent to be used is suitably determined in view of, e.g., the water-absorbing capacity of the resin to be obtained, the gel strength thereof and stability of the gel with time. It is usually 0.001 to 5.0 wt. % based on the combined amount of the monomer(s) and crosslinking agent. The amount is preferably 0.005 to 0.5 wt. % especially when the monomer is an alkali metal salt of acrylic acid, or 0.01 to 2.0 wt. % when the monomer is unneutralized acrylic acid. If the amount is less than 0.001 wt. %, a lower gel strength is likely to result, whereas if it is over 5.0 wt. %, the gel tends to become brittle on absorption of water. Thus, amounts outside the specified range are not desirable.

When required, acrylamide, acrylamide-2-methylpropanesulfonic acid salts, lower acrylic acid esters and methacrylic acid, can, e.g., be used in addition to the essential monomer(s), i.e., acrylic acid and/or acrylic acid alkali metal salt, and the crosslinking agent, such compounds being used in an amount of up to 20 wt. % based on all the monomers in view of the water-absorbing capacity, water retentivity and gel strength of the water-absorbing resin to be obtained.

According to the invention, a mixture of aqueous monomer solution containing the monomer(s) and crosslinking agent and a photoinitiator is cooled, solidified, molded and irradiated with ultraviolet rays, or a mixture of the aqueous monomer solution and a photoinitiator which evolves no gas under polymerization and crosslinking conditions is irradiated with ultraviolet rays to obtain a water-absorbing resin, which is then molded in a predetermined shape, or the mixture as held in a predetermined shape is irradiated with ultraviolet rays, whereby a molded body of water-absorbing resin is produced.

The kind of photoinitiator to be used is dependent on whether the mixture to be irradiated with ultraviolet rays is cooled and solidified.

When the mixture is cooled, solidified and irradiated with ultraviolet rays (process (2)), the kind of photoinitiator is in no way limited. It is especially desirable to use a water-soluble azo compound having an amidino group when the monomer is an alkali metal salt of acrylic acid. When the monomer is subjected to aqueous solution polymerization with ultraviolet rays, the azo compound satisfies requirements as to, e.g., the polymerization velocity, radical generating temperature and solubility in the aqueous monomer solution. Preferred examples of such azo compounds are 2,2'-azobis(N,N'-dimethyleneisobutylamidine) dihydrochloride, 2,2'-azobis(2-amidinopropane) dihydrochloride and 2,2'-azobis(N,N'-dimethyleneisobutylamidine), at least one of which is usable. The azo compound chiefly has the function of a photoinitiator but serves also as a thermal polymerization initiator. Also usable in the present invention are photoinitiators which are generally used for ultraviolet polymerization, such as diacetyl, benzoin, benzil, anthraquinone, acetophenone, diphenyl disulfide, benzophenone and derivatives of these compounds. When the monomer is acrylic acid, it is desirable to use diacetyl or like photoinitiator other than the azo compound, for example, in view of the polymerization velocity.

Alternatively when the mixture is irradiated with ultraviolet rays without cooling for solidification (process (3)), a photoinitiator is used which evolves no gas under polymerization and crosslinking conditions. More specifically, the water-soluble azo compounds among other photoinitiators mentioned above are likely to produce gas and are therefore not suited to use. In this case,therefore, it is desirable to use diacetyl and other photoinitiators which evolve no gas.

Stated more specifically, the processes (2) and (3) for producing the water-absorbing resin molded body of the present invention are practiced in the following manner.

First, specified amounts of acrylic acid and/or acrylic acid alkali metal salt, crosslinking agent and other component monomers which may be used when required are dissolved in water to prepare an aqueous monomer solution. Usually, it is desirable to adjust the monomer concentration of the solution to 25 to 65 wt. %, more desirably 30 to 60 wt. %. If the monomer concentration is less than 25 wt. %, the water-absorbing resin obtained is likely to have a lower polymerization degree, whereas if it is over 65 wt. %, the reaction system becomes heated to an excessively high temperature during reaction to produce a water-absorbing resin which is liable to be porous and less likely to have satisfactory water retentivity, hence a disadvantage.

The photoinitiator is then admixed with the aqueous monomer solution with stirring to dissolve the initiator. The amount of photoinitiator to be used is not limited specifically but is usually 0.001 to 5.0 wt. % based on the combined amount of monomers. The amount is preferably 0.01 to 1.0 wt. % when the main monomer is an acrylic acid alkali metal salt, or is preferably 0.05 to 4.0 wt. % when the main monomer is acrylic acid. For polymerization, a water-soluble polymerization initiator such as potassium persulfate can be used in combination with the photoinitiator.

In the case of the production process (2) wherein the mixture is cooled and solidified, the mixture is cooled to a temperature not higher than the solidifying point. The solidifying point, which differs, for example, with the monomer concentration, is usually up to 0°C, so that the mixture is cooled to not higher than this temperature. Preferably, the cooling temperature is -50 to -30°C.

For example in the case of the production process (2) wherein the mixture is cooled, solidified, irradiated with ultraviolet rays, the mixture can be molded by solidifying the mixture as placed in e.g. a suitable die or container, or by molding the solidified mixture in e.g. a suitable die. The mixture molded in a shape in conformity with the contemplated use is polymerized and crosslinked by irradiation with ultraviolet rays after or without removing the molding from e.g. the die. In the case of the production process (3) wherein the mixture is not cooled for solidification, the mixture is irradiated with ultraviolet rays as placed in e.g. a die or container for preparing the molding. Further when required, the molded body thus obtained can be made into various shapes by stamping, cutting or any of other methods.

The mixture is irradiated with ultraviolet rays to initiate polymerization and crosslinking reactions. Insofar as ultraviolet rays are capable of fully penetrating into the mixture, the shape of the molded mixture, die, container is not limited. When required, the irradiation with ultraviolet rays may be conducted with cooling.

To ensure convenience in executing the steps of preparing the molded product, it is desirable to use an endless belt or an open container having a large surface area.

The thickness of the solidified mixture to be irradiated with ultraviolet rays is not limited specifically. For example, even if having a thickness of not smaller than 5 cm, the mixture can be fully polymerized and crosslinked. The amount of ultraviolet rays is usually 20 to 3500 mjoules/cm$^2$ although not limited specifically. If the amount is less than this range, insufficient polymerization and crosslinking will result. Amounts exceeding the above range are undesirable since after a crosslinked polymer has been obtained, application of excessive energy is likely to break the crosslinked structure, yielding a low-molecular-weight substance and making the product feel sticky. The amount of radiation is preferably 20 to 2000 mjoules/cm$^2$. The source of ultraviolet rays to be used can be any of those already known such as mercury lamp and metal halide lamp and is suitably determined in view of the reaction conditions such as the thickness of the solidified mixture. The wavelength of radiation to be applied is usually 200 to 450 nm although not limited specifically. The application of ultraviolet rays immediately initiates the reactions.

The irradiation time is suitably determined so as to give the above-mentioned amount of radiation. For example when the endless belt is used, the mixture as placed thereon can be completely allowed to react if passed through a location, irradiated under the above condition, over a short period of time which is usually several seconds to several minutes.

These processes of the invention afford a molded body of water-absorbing resin which is substantially free from air bubbles, satisfactory in e.g. uniformity and transparency, and gives satisfactory strength to the molded body itself.

Next, a description will be given of another, less preferred process for producing a water-absorbing resin molded body.

At least one of an acrylic acid polymer and an acrylic acid alkali metal salt polymer is used said process. Examples of such polymers are those comprising acrylic acid and/or an alkali metal salt of acrylic acid as the main monomer unit and prepared by various known methods such as aqueous solution polymerization, reverse-phase emulsion polymerization and reverse-phase suspension polymerization. Useful acrylic acid alkali metal salt polymers may be those obtained by preparing an acrylic acid polymer and neutralizing the polymer with an alkali metal salt. Any of such polymers can be used suitably. The aqueous monomer solution to be used for preparing the acrylic acid polymer and/or acrylic acid alkali metal salt polymer is usually 3 to 25 wt. % in monomer concentration. The monomer concentration of the solution is adjusted preferably to 10 to 20 wt. % when the crosslinking agent to be used is a polyepoxy compound or to 3 to 15 wt. % when the agent is a polyaziridinyl compound. If the monomer concentration is lower than 3 wt. %, the water-absorbing resin obtained is likely to have a lower polymerization degree, whereas if it is in excess of 25 wt. %, the reaction system becomes heated to an excessively high temperature during the reaction and is therefore difficult to control in temperature, hence a disadvantage. The neutralization degree of the acrylic acid polymer or acrylic acid alkali metal salt polymer, the kind of alkali metal salt and the kind and amount of optional monomers like those already mentioned may be the same as in the case of the processes (2) and (3).

The viscosity of the aqueous solution of the acrylic acid polymer or acrylic acid alkali metal salt polymer obtained can be determined from the wide range of from 10 to 100000 mPa•s (cps). Especially when the crosslinking agent is a polyepoxy compound, the viscosity is preferably 30000 to 100000 mPa•s (cps), more preferably 50000 to 80000 mPa•s (cps). If the viscosity is lower than 30000 mPa•s (cps), a lower gel strength will result from crosslinking, whereas if it is over 100000 mPa•s (cps), it is difficult to remove air bubbles that would occur before crosslinking. The aqueous solution can be easily adjusted to a viscosity of 30000 to 100000 mPa•s (cps) usually by adjusting the polymer concentration to 5 to 20 wt. %. In the case where the crosslinking agent is a polyaziridinyl compound, the viscosity is preferably 10 to 50000 mPa•s (cps), more preferably 100 to 10000 mPa•s (cps). If the viscosity is lower than 10 mPa•s (cps), the crosslinking reaction results in a lower gel strength, whereas if it is over 50000 mPa•s (cps), difficulty is encountered in removing air bubbles that would occur before crosslinking. The aqueous solution can be easily adjusted to a viscosity of 10 to 50000 mPa•s (cps) usually by controlling the polymer concentration to 3 to 15 wt. %.

The kind of polyepoxy compounds and polyaziridinyl compounds for use as crosslinking agents is the same as in the processes (2) and (3). When the acrylic acid alkali metal salt polymer is used, either type of crosslinking agent is usable, while the polyaziridinyl compound is preferable to use as the crosslinking agent for the acrylic acid polymer.

The amount of crosslinking agent to be used is suitably determined in view of, i.a., the water-absorbing capacity and gel strength of the water-absorbing resin to be obtained and the stability of the gel with time. It is usually 0.05 to 5.0 parts by weight, preferably 0.5 to 3.0 parts by weight, per 100 parts by weight of the acrylic acid polymer and/or the acrylic acid alkali metal salt polymer. If the amount is less than 0. 05 part by weight, a lower gel strength is likely to result, whereas if it is over 5.0 parts by weight, a brittle gel tends to result. Thus amounts outside the specified range are undesirable.

More specifically, the above production process is practiced in the following manner.

First, an aqueous solution or mixture is prepared by mixing an acrylic acid polymer and/or an acrylic acid alkali metal salt polymer, which may usually be in the form of an aqueous solution, with at least one crosslinking agent selected from polyepoxy compounds and polyaziridinyl compounds. The polyaziridinyl compound may be diluted with, e.g., acetone before mixing. The aqueous solution or mixture is then made substantially free from air bubbles by removing air bubbles therefrom, for example, by subjecting the solution or mixture to a reduced pressure of at least 13 kPa (100 mm Hg) to below 0.1 MPa (760 mm Hg). If the pressure is lower than 13 kPa (100 mm Hg) in this case, the solution or mixture is likely to bubble up at a time and spill over the container, whereas the air bubbles are not removable at a pressure of not lower than 0.1 MPa (760 mm Hg). Alternatively, the air bubbles may be removed one by one with e.g. a syringe. It is convenient to remove the air bubbles from the mixture as placed in a molding container.

The mixture free from air bubbles and placed in the molding container is crosslinked at a temperature of 5 to 50 °C, preferably 15 to 45 °C. If the temperature is less than 5 °C, the crosslinking reaction will not progress fully, whereas if it is over 50 °C, the crosslinking reaction produces water vapor, and air bubbles occur in the molded products. Thus, temperatures outside the specified range are not desirable.

The molding container is not limited specifically but can be any of various shapes. For example, a die is favorably usable in view of convenience of handling.

In this way, a molded body of water-absorbing resin can be obtained which is substantially free from air bubbles and has high uniformity and transparency and useful strength. When required, the molded body prepared can be made into various shapes by stamping, cutting or any of other methods.

In water content, the molded bodies of water-absorbing resin obtained by the proceses (2) and (3) are usually 35 to 75 wt. %, and those prepared by the process just described are usually 80 to 97 wt. %. These bodies are generally free from air bubbles and can be handled favorably at room temperature.

Especially, the dry powder of water-absorbing resin forming the molded body obtained by the process (2) has a water-soluble content usually of 0 to 7 wt. % when the monomer is an acrylic acid alkali salt or a water-soluble content usually of 0 to 10 wt. % when the monomer is acrylic acid. Thus the value is extremely smaller than that of conventional water-absorbing resins. Accordingly, the resin has a high polymerization degree and therefore exhibits good stability with time when swollen with water and does not feel sticky.

The dry powder of water-absorbing resin forming the molded body prepared by the process described above is comparable to conventional water-absorbing resins in its capacity to absorb pure water when the monomer is an acrylic acid alkali metal salt, and is usually as high as 50 to 500 g/g in this value. On the other hand, when the monomer is acrylic acid, the resin has a lower water absorbing capacity usually of 10 to 100 g/g than when the monomer is the acrylic acid alkali metal salt, but is fully serviceable as a water-absorbing resin.

The molded body of water-absorbing resin obtained is a rubberlike elastic body and has a high gel strength. For example, the gel strength of the molded body obtained by the process (2) or (3) is 2.5 to 4 times the gel strength of water-absorbing resin as merely swollen with water when the monomer is acrylic acid, or is 4 to 8 times the latter strength when the monomer is the acrylic acid alkali metal salt. Further the gel strength of the molded body obtained by the less preferred process is 1.1 to 3 times the strength when the monomer is acrylic acid or 1.5 to 3 times the strength when the monomer is the acrylic acid alkali metal salt.

According to the invention, water-absorbing resin molded bodies of the specified structure can be obtained by the foregoing production processes.

The NMR diagnosis phantom of the present invention will be described next.

The NMR diagnosis phantom of the present invention comprises a molded body of the invention. Thus, a molded body of water-absorbing resin is produced in a desired shape as required of the NMR diagnosis phantom by one of the above processes, and is used as the phantom.

It is desirable to incorporate a paramagnetic metal and/or an alcoholic compound into the NMR diagnosis phantom of the invention since the NMR characteristics thereof can then be adjusted with extreme ease.

Paramagnetic metals are generally used as a contrast agent for NMR diagnosis. When used as a suitable concentration, the paramagnetic metal shortens T1 and T2 and acts to produce images of lesions with increased contrast. According to the present invention, therefore, the paramagnetic metal is incorporated into the phantom in the form of a water-absorbing resin molded body to shorten T1 and T2 of the phantom and make the phantom equivalent to the structure of the living body with ease.

Examples of useful paramagnetic metals are gadolinium, nickel, copper, manganese, titanium, vanadium, chromium, cobalt and iron. These paramagnetic metals are supplied in the form of water-soluble metal salts such as sulfates, nitrates, acetates and chelate compounds.

The paramagnetic metal is used in an amount of up to 10 wt. %, preferably up to 5 wt. %, based on the weight of the resin in the molded body. Amounts in excess of 10 wt. % are not desirable since T1 and T2 then become too small in comparison to the NMR data of the living structure to render the phantom unsuitable for NMR diagnosis.

The method of incorporating the paramagnetic metal into the molded body of water-absorbing resin is not limited specifically. For example, the following methods are useful. In the case of the water-absorbing resin molded body obtained by the process (2) or (3), the paramagnetic metal is incorporated into an aqueous solution for polymerizing the molded body. Alternatively, the water-absorbing resin molded body prepared is swollen with an aqueous solution containing the paramagnetic metal. In the case of the molded body obtained by the less preferred process, the paramagnetic metal is incorporated into the aqueous solution for preparing the molded body by polymerization, or into an aqueous alkali solution for neutralizing the aqueous solution of polyacrylic acid or an aqueous solution for adjusting the concentration of the acid solution. Alternatively, the molded body prepared is swollen with an aqueous solution containing the paramagnetic metal.

Furthermore, an alcoholic compound such as ethyl alcohol and glycerin which has heretofore been used as an adjusting agent for phantoms can be incorporated into the phantom of the invention, singly or in combination with the paramagnetic metal. The difference between the T1 value and the T2 value increases as the concentration of the alcoholic compound increases, so that the T1/T2 ratio of the phantom can be readily adjusted to the corresponding ratio of the living body by suitably varying the concentration.

Although the method of incorporating the alcoholic compound into the molded body of water-absorbing resin is not limited specifically, the alcoholic compound is usually added to the aqueous solution for adjusting the water content of the molded body obtained and is thereby incorporated into the molded body. The concentration of the alcoholic compound in the aqueous solution is usually up to 30 wt. %. The molded body of water-absorbing resin can be made to contain usually up to 300 wt. % of the alcoholic compound based on the weight of the resin. The amount is preferably up to 100 wt. %.

Next, the process for preparing the water-absorbing resin of the invention will be described.

In the process for preparing the water-absorbing resin of the invention, a mixture of photoinitiator and an aqueous monomer solution containing acrylic acid and/or an alkali metal salt of acrylic acid, and a crosslinking agent is cooled, solidified and thereafter irradiated with ultraviolet rays as previously stated as the process (5), whereby the water-absorbing resin can be obtained substantially free from air bubbles, with formation of low-molecular-weight substances or like water solubles greatly inhibited.

The acrylic acid and acrylic acid alkali metal salt to be used in the above process are the same as those used in the process (2) for producing the molded body of the invention. The crosslinking agent and photoinitiator to be used in the resin preparation process are the same as those for use in the molded body production process. Further the amounts of these essential components, components to be used optionally and the amounts thereof are the same as in the process (2) of the invention for producing the molded body.

The process for preparing the water-absorbing resin of the invention, which is substantially the same as the process (2) of the invention for producing the molded body, is practiced in the manner to be described in greater detail below.

First, specified amounts of acrylic acid or acrylic acid alkali metal salt, crosslinking agent and other component monomers which may be used when required are dissolved in water to prepare an aqueous monomer solution. The monomer concentration of this aqueous solution is usually 25 to 65 wt. % as in the process for producing the molded body.

Next, the photoinitiator is admixed with the aqueous monomer solution with stirring as in the case of the process for producing the molded body to dissolve the photoinitiator in the solution. The amount of photoinitiator is the same as already described, while water-soluble thermal polymerization initiators such as potassium persulfate are usable in combination therewith when required as previously stated.

With the process of the present invention for preparing the water-absorbing resin, it is essential that the mixture of aqueous monomer solution and photoinitiator be cooled, solidified and thereafter irradiated with ultraviolet rays for polymerization and crosslinking. The cooling temperature is the same as in the process for producing the molded body. The solidified mixture obtained is then placed into a suitable reactor and thereafter irradiated with ultraviolet rays for polymerization and crosslinking. The shape of the reactor for the polymerization and crosslinking reactions of the invention is not limited insofar as ultraviolet rays can fully penetrate through the reactor, whereas an endless belt or an open container having a large surface area is desirable in view of the work efficiency and convenience in performing the step of drying and pulverizing the water-absorbing resin obtained. When the endless belt is to be used, it is convenient to cool and solidify the mixture on the belt prior to ultraviolet irradiation. The thickness of the solidified mixture to be irradiated with ultraviolet rays is not limited specifically. For example, even if having a thickness of at least 5 cm, the mixture can be fully polymerized and crosslinked. Further the amount, wavelength and light source of the ultraviolet rays are, for example, the same as in the case of the process for producing the molded body. The irradiation time is suitably determined so that a predetermined amount of light is given. For example when the endless belt is used, the mixture is completely allowed to react by passing a location irradiated with ultraviolet rays under the foregoing conditions usually over a short period of time, i.e., several seconds to several minutes.

The water-absorbing resin thus obtained usually contains 35 to 75 wt. % of water and has an appearance of a transparent rubberlike elastic material. When required for the contemplated use, therefore, the resin can be dried and pulverized subsequently into particles or granules. Known methods are directly usable for these steps without necessity of using a special procedure or apparatus. Examples of useful dryers are a hot air dryer, infrared dryer and fluidized layer dryer. The drying temperature is usually 70 to 200°C. The dry water-absorbing resin obtained can be pulverized to the desired particle size using, for example, a vibrating pulverizer or impact pulverizer.

The size and shape of the water-absorbing resin obtained by the process of the invention are not limited specifically but can suitably be determined according to the contemplated use. For example, when to be used as a sanitary material, the resin is usually in the form of particles which are preferably 10 to 600 mesh in size.

The water-absorbing resin obtained by the process of the invention can be used for various uses as previously mentioned. The resin is recommendable especially for use in sanitary products, paper diapers and like sanitary goods. Depending on the use, the water-absorbing resin is used singly or in combination with an inorganic powder such as silicon dioxide powder or titanium oxide powder, or organic filler such as rubber. Examples of useful silicon dioxide powders are colloidal silica, white carbon and superfine particulate silica.

The water-absorbing resin pulverized by the above method can be thereafter modified when so required. For example, the surface of the pulverized water-absorbing resin can be modified by reacting the carboxylate present in the resin with a known crosslinking agent such as a polyepoxy compound which is typically a water-soluble diglycidyl ether compound, aldehyde compound or polyvalent metal salt. The modified product is usable also for the same use as mentioned above.

The dry powder of water-absorbing resin prepared by the foregoing production process of the invention usually has 0 to 7 wt. % of water solubles content, which is much smaller than is the case with the conventional water-absorbing resins. This indicates a high polymerization degree, which gives good stability to the resin when it is swollen with water and makes the resin feel nonsticky. The resin is comparable to conventional water-absorbing resins in ability to absorb pure water, which is usually as high as 50 to 500 g/g.

The present invention has the following remarkable advantages.

(i) The invention provides for the first time a novel molded body of water-absorbing resin which fully fulfills the requirements of being excellent in water-absorbing ability and uniformity and is satisfactory in transparency, having strength and being free from air bubbles. The molded body is well suited as a molding which swells with water for use in the field of materials for medical phantoms and toys.

(ii) The processes of the invention readily provides the novel molded body having the above-mentioned outstanding characteristics. The molded body can be formed with extreme ease especially by the process (2) because the mixture is once cooled to not higher than the solidifying point.

(iii) The production process (3) permits molding of delicate portions when practiced with a properly selected molding container, and is therefore suited to the production of molded bodies of water-absorbing resin having delicate portions.

(iv) The phantom of the invention, which is in the form of a water-absorbing resin molded body, can be swollen with water and thereby adjusted to a suitable proton concentration (water content). Further a phantom of desired configuration can be readily prepared in conformity with the contemplated use (e.g., a particular organ).

(v) The gel of water-absorbing resin forming the phantom has a sufficient strength for the use of the phantom and will not deform with the lapse of time. The water-absorbing resin forming the phantom has good uniformity, is free from air bubbles and therefore provides sharp NMR images. The gel has NMR characteristics of good stability as a phantom and remains free of variations in T1 and T2. The resin is stable at room temperature and is therefore easy to handle.

EP 0 481 087 B1

(vi) The phantom in the form of a water-absorbing resin molded body is usable as it is when the NMR data thereof is equivalent to the NMR data of the living structure, whereas if T1 and T2 of the phantom differ from those of the living structure, the T1 and T2 of the phantom can be readily made equivalent to those of the living structure by incorporating a paramagnetic metal and/or an alcoholic compound into the water-absorbing resin.

(vii) The process of the invention for preparing water-absorbing resin provides a water-absorbing resin substantially free from air bubbles and greatly reduced in the contents of low-molecular-weight substances or like water solubles without in any way impairing the inherent properties of the resin such as a water-absorbing capacity. The water-absorbing resin, which has a high polymerization degree, retains good stability with time when swollen with water and does not feel sticky because the resin is almost free from low-molecular-weight substances.

The present invention will be described in greater detail with reference to the following examples, comparative examples and preparation examples.

I. Preparation of water-absorbing resin molded bodies by production processes (2) and (3)

Example 1

To 200 g of acrylic acid and 251 g of water was added 83 g (corresponding to 75 mole % based on acrylic acid) of sodium hydroxide with ice-cooling for neutralization. N,N'-Methylenebisacrylamide (hereinafter referred to as "MBAM") was dissolved in an amount of 0.02 g (0.008 wt. % based on the combined amount of the monomers) in the solution, and nitrogen gas was thereafter introduced into the resulting solution to expell dissolved oxygen. With the solution adjusted to a temperature of 10°C, 2,2'-azobis(N,N'-dimethyleneisobutylamidine) dihydrochloride (product of WAKO PURE CHEMICAL INDUSTRIES, LTD., brand name: "VA-044") was further dissolved in an amount of 0.08 g (0.032 wt. % based on the combined amount of all monomers) in the solution to obtain an adjusted solution $\underline{a}$ containing the monomers in a combined concentration of 46 wt. %.

The adjusted solution $\underline{a}$ (200 g) was placed into a glass dish, 15 cm in inside diameter and 3 cm in depth, and allowed to stand in a constant-temperature chamber at -20°C for 1 hour for solidification to obtain a molded monomer.

The molded monomer was irradiated with ultraviolet rays by a device (produced by EYE GRAPHIC CO., LTD. and having two 2-KW high-pressure mercury lamps, 80 W/cm, wavelength 250 nm) in an amount of 900 mjoules/cm$^2$ for 5 minutes to obtain a molded body of water-containing gel crosslinked copolymer (water-absorbing resin) having a thickness of 1.0 cm and rubberlike elasticity. The molded body was dried by a hot air dryer at 160°C for 12 hours and thereafter pulverized into a powder of about 60 mesh by a vibrating pulverizer to obtain a powdery water-absorbing resin.

The molded body obtained was checked for air bubbles, transparency and strength. The powdery water-absorbing resin was checked for water-soluble content and water-absorbing capacity. Table 1 shows the result.

Examples 2-19 and Comparative Examples 1-4

Molded bodies of water-containing gel crosslinked copolymer (water-absorbing resin) and powdery water-absorbing resins were prepared in the same manner as in Example 1 with the exception of using the crosslinking agent, photoinitiator and adjusted solution $\underline{a}$ listed in Table 1 in the listed amounts and also using the freezing condition and irradiation condition given in Table 1 .

The molded bodies obtained were checked for air bubbles, transparency and strength. The powdery water-absorbing resins were also checked for water-solubles content and water-absorbing capacity. Table 1 shows the results.

Example 20

With ice-cooling, 235 g of water was added to 200 g of acrylic acid, 0.1 g (0.05 wt. % based on the combined amount of the monomers) of MBAM was dissolved in the solution, and nitrogen gas was thereafter introduced into the resulting solution to drive out dissolved oxygen. With the solution adjusted to a temperature of 10°C, "Darocur® 1173" (MERCK of Germany) was added thereto in an amount of 0.6 g (0.3 wt. % based on the combined amount of monomers) to obtain an adjusted solution $\underline{b}$ containing the monomers in a combined concentration of 46 wt. %.

11

In the same manner as in Example 1, the adjusted solution b afforded a molded body of water-absorbing resin, i.e., water-containing gel crosslinked copolymer and powdery water-absorbing resin.

The molded body obtained was checked for air bubbles, transparency and strength. The powdery water-absorbing resin was further checked for water-soluble content and water-absorbing capacity. Table 1 shows the result.

Examples 21-24

Molded bodies of water-containing gel crosslinked copolymer (water-absorbing resin) and powdery water-absorbing resins were prepared in the same manner as in Example 20 with the exception of using the crosslinking agent, photoinitiator and adjusted solution b listed in Table 1 in the listed amounts and also using the freezing condition and irradiation condition given in Table 1.

The molded bodies obtained were checked for air bubbles, transparency and strength. The powdery water-absorbing resins were also checked for water-solubles content and water-absorbing capacity. Table 1 shows the results.

Comparative Example 5

The powder water-absorbing resin obtained in Example 1 was placed into the same glass dish as used in Example 1 and swollen with water to obtain a water-containing gel resembling a molded body.

The gel obtained was checked for air bubbles, transparency and strength, with the result shown in Table 1.

Formation of air bubbles, water-absorbing capacity, water-solubles content, transparency and strength were determined by the following methods.

Formation of air bubbles:

The molded body obtained or the water-containing gel of Comparative Example 5 was checked for air bubbles with the unaided eye according to the following criteria.

A:    No air bubbles.
B:    Up to 3 air bubbles, not larger than about 0.1 mm in diameter, per 1 $cm^3$.
C:    Numerous air bubbles.

Water-absorbing capacity:

Expressed as the amount of pure water absorbed by the dry powdery water-absorbing resin per gram thereof.

Water-solubles content:

Distilled water (300 ml) was placed into a 500-ml Erlenmeyer flask, 0.2 ± 0.02 g of dry powdery water-absorbing resin accurately weighed out was added to the water with stirring, and the mixture was thereafter allowed to stand in the dark for 24 hours. The mixture was then filtered in a vacuum using glass fiber filter paper of MILLIPORE LTD. The filtrate was heated at 105°C for 18 hours in a dryer, the resulting residual content was measured, and the water-solubles content was calculated from the following equation.

Water-solubles content = (residual content x 100)/weight of sample

Transparency:

The molded body obtained or the water-containing gel of Comparative Example 5 was swollen with water to a water content of 70% and then made into a 1-cm-thick test piece, which was thereafter placed on paper bearing Arabic numerals printed with 14-point types. The test piece was checked as to whether the Arabic numerals were legible with the unaided eye according to the following criteria.

A:    High transparency, and all the Arabic numerals were legible.
B:    The numerals were difficult to read owing to air bubbles.
C:    Totally illegible.

Strength:

The molded body obtained or the water-containing gel of Comparative Example 5 was swollen with pure water to a water content of 70% and then tested for strength using Neo Curdmeter®, product of IIO ELECTRIC CO.

Table 1

| Example | Neutralization degree of acrylic acid (mole %) | Crosslinking agent (g) | | Catalyst (g) | | Adjusted solution (g) |
|---|---|---|---|---|---|---|
| Example 1 | 75 | MBAM | 0.02 | VA-044 | 0.08 | a200 |
| " 2 | " | " | 0.02 | " | 0.08 | a200 |
| " 3 | " | " | 0.04 | " | 0.08 | a200 |
| " 4 | " | " | 0.02 | " | 0.16 | a400 |
| " 5 | " | " | 0.04 | " | 0.16 | a400 |
| " 6 | " | " | 0.04 | " | 0.08 | a200 |
| " 7 | " | " | 0.02 | " | 0.08 | a200 |
| " 8 | " | " | 0.02 | Darocur® 1173 | 0.025 | a200 |
| " 9 | " | " | 0.02 | " | 0.025 | a200 |
| " 10 | " | " | 0.04 | " | 0.025 | a200 |
| " 11 | " | " | 0.04 | " | 0.05 | a400 |
| " 12 | " | EPOLIGHT® 400E | 0.10 | VA-044 | 0.08 | a200 |
| " 13 | " | TAZO® | 0.01 | " | 0.08 | a200 |
| " 14 | 50 | MBAM | 0.02 | Darocur® 1173 | 0.1 | a200 |
| " 15 | 25 | " | 0.02 | " | 0.2 | a200 |

Table 1 (continued)

| | Neutralization degree of acrylic acid (mole %) | Crosslinking agent (g) | | Catalyst (g) | | Adjusted solution (g) |
|---|---|---|---|---|---|---|
| Example 16 | 75 | MBAM | 0.02 | Darocur® 1173 | 0.025 | a200 |
| " 17 | " | " | 0.04 | " | 0.025 | a200 |
| " 18 | " | " | 0.04 | " | 0.05 | a400 |
| " 19 | 50 | " | 0.02 | " | 0.1 | a200 |
| " 20 | 0 (unneu-tralized) | " | 0.1 | " | 0.6 | b200 |
| " 21 | " | " | 0.2 | " | 1.2 | b400 |
| " 22 | " | EPOLIGHT® 400E | 0.5 | " | 0.6 | b200 |
| " 23 | " | TAZO® | 0.05 | " | 0.6 | b200 |
| " 24 | " | MBAM | 0.1 | " | 0.6 | b200 |
| Comp. Ex. 1 | 75 | " | 0.02 | VA-044 | 0.08 | a200 |
| " 2 | " | " | 0.08 | " | 0.08 | a200 |
| " 3 | " | " | 0.04 | " | 0.08 | a400 |
| " 4 | " | " | 0.02 | " | 0.16 | a200 |
| " 5 | " | " | 0.02 | " | 0.08 | -- |

EP 0 481 087 B1

Table 1 (continued)

| | | Freezing condition (°C, hr) | Irradiation condition (mj/$cm^2$) | Air bubbles | Water-absorbing capacity (g/g) | Water-solubles content (wt. %) | Trans-parency | Strength (dynes/$cm^2$) |
|---|---|---|---|---|---|---|---|---|
| Example | 1 | -20, 1 | 900 | A | 285 | 2.5 | A | $11 \times 10^4$ |
| " | 2 | -40, 1 | 900 | A | 270 | 1.3 | A | $17 \times 10^4$ |
| " | 3 | -20, 1 | 900 | A | 125 | 0.8 | A | $26 \times 10^4$ |
| " | 4 | -20, 3 | 900 | A | 412 | 5.3 | A | $8 \times 10^4$ |
| " | 5 | -40, 3 | 900 | A | 398 | 4.0 | A | $11 \times 10^4$ |
| " | 6 | -20, 1 | 1800 | A | 159 | 1.3 | A | $20 \times 10^4$ |
| " | 7 | -40, 1 | 1800 | A | 297 | 3.8 | A | $12 \times 10^4$ |
| " | 8 | -20, 1 | 900 | A | 265 | 4.3 | A | $12 \times 10^4$ |
| " | 9 | -40, 1 | 900 | A | 248 | 3.1 | A | $17 \times 10^4$ |
| " | 10 | -20, 1 | 900 | A | 97 | 1.9 | A | $24 \times 10^4$ |
| " | 11 | -40, 3 | 900 | A | 295 | 5.2 | A | $14 \times 10^4$ |
| " | 12 | -20, 1 | 900 | A | 303 | 3.7 | A | $10 \times 10^4$ |
| " | 13 | -20, 1 | 900 | A | 253 | 2.1 | A | $14 \times 10^4$ |
| " | 14 | -20, 1 | 900 | A | 135 | 4.9 | A | $9 \times 10^4$ |
| " | 15 | -20, 1 | 900 | A | 63 | 6.4 | A | $8 \times 10^4$ |

Table 1 (continued)

| | Freezing condition (°C, hr) | Irradiation condition (mj/cm$^2$) | Air bubbles | Water-absorbing capacity (g/g) | Water-solubles content (wt. %) | Trans-parency | Strength (dynes/cm$^2$) |
|---|---|---|---|---|---|---|---|
| Example 16 | No freezing | 900 | A | 317 | 17.6 | A | $11 \times 10^4$ |
| " 17 | No freezing | 900 | A | 185 | 12.3 | A | $18 \times 10^4$ |
| " 18 | No freezing | 900 | A | 385 | 19.2 | A | $11 \times 10^4$ |
| " 19 | No freezing | 900 | A | 151 | 18.8 | A | $9 \times 10^4$ |
| " 20 | -20, 1 | 900 | A | 28 | 8.6 | A | $6 \times 10^4$ |
| " 21 | -20, 3 | 900 | A | 31 | 8.6 | A | $6 \times 10^4$ |
| " 22 | -20, 1 | 900 | A | 35 | 9.3 | A | $6 \times 10^4$ |
| " 23 | -20, 1 | 900 | A | 17 | 7.4 | A | $7 \times 10^4$ |
| " 24 | No freezing | 900 | A | 44 | 18.5 | A | $6 \times 10^4$ |
| Comp. Ex. 1 | No freezing | 900 | C | 301 | 15.3 | B | $8 \times 10^4$ |
| " 2 | No freezing | 900 | C | 95 | 11.7 | B | $8 \times 10^4$ |
| " 3 | No freezing | 900 | C | 453 | 19.8 | B | $7 \times 10^4$ |
| " 4 | No freezing | 1800 | C | 328 | 19.0 | B | $7 \times 10^4$ |
| " 5 | -- | -- | D | 285 | 2.5 | C | $2.4 \times 10^4$ |

In the above table, "Darocur® 1173" (brand name of MERCK) stands for 2-hydroxy-2-methyl-1-phenylpropane-1-one, "EPOLIGHT® 400E" (brand name of KYOEISHA CHEMICAL CO., LTD.) for polyethylene glycol diglycidyl ether, and "TAZO®" (brand name of SOGO YAKKO CO., LTD) for tetramethylolmethane-tri-β-aziridinyl)propionate.

16

The symbol D in the column of "Air bubbles" indicates that air bubbles are present between particles of swollen water-absorbing resin.

II. Preparation of water-absorbing resin molded bodies by the less preferred production process

Preparation Example 1

Preparation of acrylic acid polymer and polymer of acrylic acid alkali metal salt

A 200 g quantity of acrylic acid and 1030 g of deionized water were placed into a 2-liter flask equipped with a stirrer, thermometer, condenser and nitrogen supply tube under a nitrogen atmosphere. The solution was heated to 60°C, and a solution of 0.73 g of ammonium persulfate in 2 g of deionized water was added to the solution to initiate polymerization with cooling. Upon the temperature of the solution rising to 70°C owing to the heat of polymerization, 80 g of deionized water was further added so as to control the solution to a maximum temperature of 90 to 95°C. When the temperature of the solution dropped, the supply of nitrogen was discontinued to effect polymerization. Two hours thereafter, a solution of 0.025 g of hydroquinone in 1 g of deionized water was added to the reaction mixture to complete the polymerization.

To 1314 g of the resulting aqueous solution of acrylic acid polymer (adjusted solution c) was added a solution of 82 g of sodium hydroxide in 216 g of deionized water to obtain an aqueous solution of polymer of acrylic acid sodium salt (adjusted solution d) having a nonvolatiles content of 15 % and a neutralization degree of 75%.

Water (1409 g) was added to the adjusted solution d to obtain an aqueous solution of polymer of acrylic acid sodium salt (adjusted solution e) having a nonvolatiles content of 8% and a neutralization degree of 75%.

Sodium hydroxide (55 g) dissolved in 145 g of deionized water was added to 1314 g of the adjusted solution c to obtain an aqueous solution of polymer of acrylic acid sodium salt (adjusted solution f) having a nonvolatiles content of 15% and a neutralization degree of 50%.

Example 25

Into a cylindrical container made of glass and measuring 20 cm in diameter and 10 cm in depth were placed 1570 g of the adjusted solution d and 4.71 g of polyethylene glycol diglycidyl ether (brand name: EPOLIGHT® 400E, product of KYOEISHA CHEMICAL CO., LTD., the amount of the ether being 2% based on the combined amount of all monomers). The mixture was stirred and thereafter allowed to stand at 10°C and 40 kPa (300 mm Hg) for 24 hours to remove air bubbles. Subsequently, the mixture was allowed to stand in a constant-temperature chamber at 40°C for 3 days to obtain a molded body of water-absorbing resin, i.e., water-containing gel crosslinked polymer, in the form of a solid cylinder measuring 20 cm in diameter and 5 cm in length.

Examples 26-30

Molded bodies of water-absorbing resin, i.e., water-containing gel crosslinked polymer, were prepared in the same manner as in Example 25 with the exception of using the adjusted solution and the crosslinking agent listed in Table 2 in the listed amounts under the crosslinking condition given in the table.

Comparative Example 6

The powdery water-absorbing resin obtained in Example 1 was swollen with water in the same manner as in Comparative Example 5 to a water content of 95%.

The bodies obtained in Examples 25 to 30 and Comparative Example 6 were checked for air bubbles, transparency and strength. Powdery water-absorbing resins were prepared from the respective bodies and checked for water-absorbing capacity.

Table 2 below shows the results.

The products were checked for air bubbles, water absorbing capacity, transparency and strength in the same manner as already described. The transparency and strength were determined with the water-containing gel made to contain 95% of water.

17

Table 2

| | Adjusted solution (amount, g) | | Crosslinking agent (amount, g) | | Deaeration pressure, $kPa$ (mm Hg) | Crosslinking | |
|---|---|---|---|---|---|---|---|
| | | | | | | Time (days) | Temp.(°C) |
| Example 25 | d | 1570 | EPOLIGHT® 400E | 4.71 | 40 (300) | 3 | 40 |
| "    26 | d | 2355 | " | 7.07 | 40 (300) | 5 | 40 |
| "    27 | d | 1570 | " | 7.07 | 40 (300) | 3 | 40 |
| "    28 | e | 1570 | TAZO®* | 2.51 | 40 (300) | 1 | 20 |
| "    29 | c | 1570 | " | 2.51 | 40 (300) | 3 | 20 |
| "    30 | f | 1570 | " | 7.07 | 40 (300) | 5 | 40 |

| | Air bubbles | Water-absorbing capacity (g/g) | Transparency | Gel strength (dynes/cm$^2$) |
|---|---|---|---|---|
| Example 25 | A | 307 | A | $3.2 \times 10^4$ |
| "    26 | A | 312 | A | $2.9 \times 10^4$ |
| "    27 | A | 275 | A | $3.7 \times 10^4$ |
| "    28 | A | 243 | A | $4.5 \times 10^4$ |
| "    29 | A | 26 | A | $2.3 \times 10^4$ |
| "    30 | A | 285 | A | $2.5 \times 10^4$ |
| Comp.Ex. 6 | C | 116 | C | $2.0 \times 10^4$ |

Note        * 5% acetone solution

EP 0 481 087 B1

III. Preparation of phantoms for NMR diagnosis

Example 31

The molded body obtained in Example 1 was placed into a polyethylene bag, into which the mixture of 434 ml of physiological saline and 0.54 g of meglumine gadopentetate (1.15 ml of "Magnevist®," brand name for product of SCHERING of Germany) was placed to cause the molded body to absorb the solution. The bag was sealed off and then allowed to stand in the dark for 2 days, whereby a phantom of the invention for NMR diagnosis was obtained which contained 90% of water (inclusive of the solutes) and 1 wt. % of meglumine gadopentetate based on the weight of water-absorbing resin in the molded body.

The phantom was installed in an NMR diagnosis apparatus and checked for spin-lattice (longitudinal) relaxation time (T1) and spin-spin (transverse) relaxation time (T2). Table 3 shows the result.

Example 32-40

Phantoms of the invention for NMR diagnosis were prepared in the same manner as in Example 31 except for changes in the kind and amount of paramagnetic metal, amount of saline, water content and duration of standing of the sealed polyethylene bag as listed in Table 3.

The phantoms were checked for T1 and T2 in the same manner as in Example 31. Table 3 shows the results.

Example 41

A phantom of the invention for NMR diagnosis was prepared in the same manner as in Example 31 with the exception of using 4.4 g of aqueous ethanol solution (ethanol concentration 1 wt. %) in addition to 434 ml of physiological saline and meglumine gadopentetate. The molded body contained 8 wt. % of ethanol based on the water-absorbing resin therein.

The phantom was checked for T1 and T2 in the same manner as in Example 31. Table 3 shows the result.

Examples 42-44

Phantoms of the invention for NMR diagnosis were prepared in the same manner as in Example 41 except changes in the kind and amount of alcoholic compound used, amount of saline, water content and duration of standing of the sealed polyethylene bag as listed in Table 3.

The phantoms obtained were checked for T1 and T2 in the same manner as in Example 31. Table 3 shows the results.

Examples 45-49

Phantoms of the invention for NMR diagnosis were prepared in the same manner as in Example 31 with the exception of changing the type of molded body and duration of standing of the sealed polyethylene bag as listed in Table 3.

The phantoms obtained were checked for T1 and T2 in the same manner as in Example 31. Table 3 shows the results.

EP 0 481 087 B1

Table 3

| Ex. | Type of molded body | Paramagnetic metal supplying compound (amount) | | Alcoholic compound (amount) | | Amount of saline (ml) |
|---|---|---|---|---|---|---|
| 31 | Ex. 1 | M.G.* | 1.0% | -- | -- | 434 |
| 32 | " 1 | " | 2.0% | -- | -- | 433 |
| 33 | " 1 | " | 0.5% | -- | -- | 434.3 |
| 34 | " 1 | " | 1.0% | -- | -- | 434 |
| 35 | " 1 | " | 1.0% | -- | -- | 253.3 |
| 36 | " 1 | " | 1.0% | -- | -- | 130.6 |
| 37 | " 1 | -- | -- | -- | -- | 434.5 |
| 38 | " 1 | -- | -- | -- | -- | 434.5 |
| 39 | " 1 | Copper sulfate | 1.0% | -- | -- | 434.5 |
| 40 | " 1 | Nickel sulfate | 1.0% | -- | -- | 434.5 |
| 41 | " 1 | M.G.* | 1.0% | 1.0% Ethanol | 8% | 434 |
| 42 | " 1 | " | 1.0% | 5.0% Ethanol | 40% | 434 |
| 43 | " 1 | " | 1.0% | 5.0% Ethanol | 40% | 434 |
| 44 | " 1 | " | 1.0% | 5.0% Glycerin | 40% | 434 |
| 45 | " 3 | " | 1.0% | -- | -- | 434 |
| 46 | " 8 | " | 1.0% | -- | -- | 434 |
| 47 | " 16 | " | 1.0% | -- | -- | 434 |
| 48 | " 16 | " | 1.0% | -- | -- | 434 |
| 49 | " 20 | " | 1.0% | -- | -- | 434 |

Note        * M.G.        meglumine gadopentetate

20

Table 3 (continued)

| Example | Duration of standing after sealing (days) | Water content (%) | $T_1$ (msec) | $T_2$ (msec) |
|---|---|---|---|---|
| 31 | 2 | 90 | 875 | 128 |
| 32 | 2 | 90 | 653 | 93 |
| 33 | 2 | 90 | 987 | 185 |
| 34 | 90 | 90 | 868 | 128 |
| 35 | 2 | 85 | 629 | 87 |
| 36 | 2 | 77.3 | 536 | 45 |
| 37 | 2 | 90 | 1138 | 285 |
| 38 | 90 | 90 | 1130 | 283 |
| 39 | 2 | 90 | 912 | 153 |
| 40 | 2 | 90 | 907 | 144 |
| 41 | 2 | 90 | 863 | 101 |
| 42 | 2 | 90 | 860 | 54 |
| 43 | 90 | 90 | 855 | 52 |
| 44 | 2 | 90 | 842 | 47 |
| 45 | 2 | 90 | 844 | 113 |
| 46 | 2 | 90 | 889 | 137 |
| 47 | 2 | 90 | 921 | 169 |
| 48 | 90 | 90 | 917 | 167 |
| 49 | 2 | 90 | 915 | 134 |

Example 50

To 1314 g of the adjusted solution c obtained in Preparation Example 1 were added 82 g of sodium hydroxide and 2.47 g of meglumine gadopentetate which were dissolved in 1079 g of deionized water to prepare an aqueous solution of sodium polyacrylate having a nonvolatiles content of 10% and a neutralization degree of 75%.

Into a glass container, 20 cm in diameter and 10 cm in depth, were placed 1638 g of the aqueous solution of sodium polyacrylate and 4.91 g of polyethylene glycol diglycidyl ether (brand name : "EPOLIGHT® 400E," product of KYOEISHA CHEMICAL CO., LTD.), which were stirred and then allowed to stand at 20°C for 3 hours. After removing air bubbles from the surface of the mixture with a syringe, the mixture was allowed to stand at 10°C and 40 kPa (300 mm Hg) for 24 hours to further remove air bubbles. The mixture was thereafter allowed to stand as placed in a constant-temperature chamber at 40°C for 3 days to obtain a phantom of the invention for NMR diagnosis, 20 cm in diameter and 5 cm in length.

When the phantom (solid cylindrical molded body) was checked for air bubbles and transparency, the result was A for each, and the phantom was $3.4 \times 10^4$ dynes/cm$^2$ in strength.

The phantom was also checked for T1 and T2 in the same manner as in Example 31. Table 4 shows the result.

Examples 51-53

Phantoms of the invention for NMR diagnosis were prepared in the same manner as in Example 50 with the exception of changing the amount of paramagnetic metal supplying compound used, the amount of deionized water added for neutralization, water content and duration of standing of the sealed polyethylene bag as listed in Table 4.

The phantoms obtained were checked for T1 and T2 in the same manner as in Example 31. Table 4 also shows the results.

Example 54

A phantom of the invention for NMR diagnosis was prepared in the same manner as in Example 50 except that 54 g of ethanol was added to the deionized water used for neutralization.

The phantom obtained was checked for T1 and T2 in the same manner as in Example 31. Table 4 shows the result.

Example 55

To 1314 g of the adjusted solution c obtained in Preparation Example 1 was added 1.97 g of meglumine gadopentetate dissolved in 1150 g of deionized water to prepare an aqueous solution of polyacrylic acid having a nonvolatiles content of 8%.

The aqueous solution of polyacrylic acid (1638 g) was placed into a glass container, 20 cm in diameter and 10 cm in depth, 2.62 g of TAZO® dissolved in 49.78 g of acetone was then slowly added to the solution, the mixture was then allowed to stand at 10°C for 1 hour, and the air bubbles remaining on the surface were removed with a syringe. The mixture was thereafter allowed to stand at 10°C and 40 kPa (300 mm Hg) for 24 hours to remove air bubbles. Subsequently, the mixture was allowed to stand as placed in a constant-temperature chamber at 20°C for 3 days to obtain a phantom of the invention, 20 cm in diameter and 5 cm in length, for NMR diagnosis.

When the phantom (solid cylindrical molded body) was checked for air bubbles and transparency, the result was A for each, and the strength of the phantom $2.6 \times 10^4$ dynes/cm$^2$.

The phantom obtained was also checked for T1 and T2 in the same manner as in Example 31. Table 4 shows the result.

Table 4

| Ex. | Paramagnetic metal supplying compound (amount) | | Alcoholic compound (amount) | | Amount of deionized water for neutral-ization(ml) |
|---|---|---|---|---|---|
| 50 | M.G.* | 1.0% | -- | -- | 1079 |
| 51 | " | 2.0% | -- | -- | 1076.5 |
| 52 | " | 1.0% | -- | -- | 253 |
| 53 | " | 1.0% | -- | -- | 1079 |
| 54 | " | 1.0% | 5.0% Ethanol | 21.9% | 1079 |
| 55 | " | 1.0% | -- | -- | 1150 |

| Ex. | Duration of standing (days) | Water content (%) | $T_1$ (msec) | $T_2$ (msec) |
|---|---|---|---|---|
| 50 | 0 | 90 | 1230 | 359 |
| 51 | 0 | 90 | 729 | 216 |
| 52 | 0 | 85 | 972 | 255 |
| 53 | 90 | 90 | 1220 | 355 |
| 54 | 0 | 90 | 1207 | 108 |
| 55 | 0 | 92 | 1411 | 486 |

Note    * M.G.    meglumine gadopentetate

Example 56

Each of the NMR diagnosis phantoms obtained in Examples 31 to 54 was placed on a horizontal plate (3 mm in thickness) of acrylic resin provided in the static magnetic field (0.15T) of an NMR diagnosis apparatus, and was photographed vertically from above. An approximately perfect square image was produced from each of the phantoms and had uniform luminance over the entire area.

Example 57

To check the NMR diagnosis phantoms obtained in Examples 31, 34, 41, 47 and 50 for uniformity, the gel used for each phantom was randomly cut to prepare solid cylinders measuring 7.5 mm in diameter and 1 cm in height. The cylinders were packed into the sample tube (7.5 cm in diameter) of a microanalysis NMR spectrometer (0.47T) to measure the amount of protons by the 90-deg pulse method, T2 by the CPMG method and T1 by the IR method at 20°C. A plate of the gel was also used to obtain like measurements at the central and peripheral ten portions of the plate. The measurements obtained were 89.5 to 90.2% for water content, 868 to 879 msec for T1 and 126 to 131 msec for T2, indicating that the water-containing gel of the invention was uniform in respect of NMR data. Since an accurate proper image was produced from the homogeneous gel plate with the above-mentioned NMR diagnosis apparatus, the above results also indicate that the imaging system of the apparatus was adjusted and maintained satisfactorily.

Comparative Example 7

The same powdery water-absorbing resin as used in Comparative Example 5, i.e., sodium polyacrylate, was placed into a glass container, 10 cm in length, 10 cm in width and 3 cm in depth. The resin was swollen with physiological saline to a water content of 90% to obtain a water-containing gel resembling a molding.

Next, the gel was placed on the acrylic resin horizontal plate (3 mm in thickness) in the static magnetic field (0.15T) of the NMR diagnosis apparatus and was photographed vertically from above. However, no sharp image was obtained, while the luminance of the image formed was not uniform over the entire image area.

**Claims**

1. A molded body of a water-absorbing resin characterized in that the water-absorbing resin is a crosslinked polymer of acrylic acid or a crosslinked polymer of an acrylic acid alkali metal salt and forms a uniform matrix phase wherein the molecules of the resin are interlocked with one another, and water absorbed by the resin is uniformly dispersed as water chemically and/or physically bonded to the resin, and that only up to three air bubbles, not larger than 0.1 mm in diameter, may be present per $cm^3$ of said molded body.

2. A molded body as defined in claim 1 wherein the water-absorbing resin is the crosslinked polymer of an acrylic acid alkali metal salt and has a water-solubles content of 0 to 7 wt.-% and a capacity to absorb 50 to 500 g/g of pure water when dry.

3. A process for producing the molded body defined in claim 1, characterized by cooling, solidifying and molding a mixture of a photoinitiator and an aqueous monomer solution containing acrylic acid and/or an alkali metal salt of acrylic acid, and a crosslinking agent, and irradiating the molded mixture with ultraviolet rays to polymerize and crosslink the mixture.

4. A process as defined in claim 3 wherein the crosslinking agent is at least one compound selected from divinyl compounds, polyepoxy compounds and polyaziridinyl compounds.

5. A process for producing the molded body defined in claim 1, characterized by irradiating a mixture of a photoinitiator evolving no gas under polymerization and crosslinking conditions and an aqueous monomer solution containing acrylic acid and/or an alkali metal salt of acrylic acid and a crosslinking agent with ultraviolet rays for polymerization and crosslinking to obtain a water-absorbing resin, and molding the resin into a predetermined shape, or by irradiating the mixture with ultraviolet rays for polymerization and crosslinking while holding the mixture in a predetermined shape.

6. A process as defined in claim 5 wherein the crosslinking agent is at least one compound selected from divinyl compounds, polyepoxy compounds and polyaziridinyl compounds.

7. A phantom for NMR diagnosis characterized in that the phantom comprises the molded body defined in claim 1.

8. A phantom for NMR diagnosis as defined in claim 7 which contains a paramagnetic metal and/or an alcoholic compound.

9. A process for producing a water-absorbing resin characterized by cooling and solidifying a mixture of a photoinitiator and an aqueous monomer solution containing an alkali metal salt of acrylic acid and a crosslinking agent, and irradiating the solidified mixture with ultraviolet rays to polymerize and crosslink the mixture.

10. A process as defined in claim 9 wherein the crosslinking agent is at least one compound selected from divinyl compounds, polyepoxy compounds and polyaziridinyl compounds.

**Patentansprüche**

1.  Formkörper aus einem Wasser-absorbierenden Harz, dadurch gekennzeichnet, daß das Wasser-absorbierende Harz ein vernetztes Polymer von Acrylsäure oder ein vernetztes Polymer eines Acrylsäure-Alkalimetallsalzes ist und eine einheitliche Matrixphase bildet, in der die Moleküle des Harzes ineinandergeschachtelt sind und durch das Harz absorbiertes Wasser gleichmäßig als Wasser, das chemisch und/oder physikalisch an das Harz gebunden ist, dispergiert ist, und daß nur bis zu drei Luftblasen mit einem Durchmesser, der nicht größer als 0,1 mm ist, pro $cm^3$ des Formkörpers anwesend sein können.

2.  Formkörper wie in Anspruch 1 definiert, in welchem das Wasser-absorbierende Harz das vernetzte Polymer eines Acrylsäure-Alkalimetallsalzes ist und einen Gehalt an in Wasser löslichen Stoffen von 0 bis 7 Gew.-% und im trockenen Zustand eine Absorptionsfähigkeit für reines Wasser von 50 bis 500 g/g aufweist.

3.  Verfahren zur Herstellung des in Anspruch 1 definierten Formkörpers, gekennzeichnet durch Abkühlen, Verfestigen und Formen einer Mischung eines Photoinitiators und einer wäßrigen Monomer-Lösung, die Acrylsäure und/oder ein Alkalimetallsalz von Acrylsäure und ein Vernetzungsmittel enthält, und die Bestrahlung der geformten Mischung mit UV-Strahlen zwecks Polymerisation und Vernetzung der Mischung.

4.  Verfahren wie in Anspruch 3 definiert, in welchem das Vernetzungsmittel mindestens eine Verbindung ist, die aus Divinyl-Verbindungen, Polyepoxy-Verbindungen und Polyaziridinyl-Verbindungen ausgewählt ist.

5.  Verfahren zur Herstellung des in Anspruch 1 definierten Formkörpers, gekennzeichnet durch Bestrahlung einer Mischung eines unter Polymerisations- und Vernetzungs-Bedingungen kein Gas entwickelnden Photoinitiators und einer wäßrigen Monomer-Lösung, die Acrylsäure und/oder ein Alkalimetallsalz von Acrylsäure und ein Vernetzungsmittel enthält, mit UV-Strahlen zwecks Polymerisation und Vernetzung unter Erhalt eines Wasser-absorbierenden Harzes und das Formen des Harzes in eine vorher festgelegte Gestalt, oder durch Bestrahlung der Mischung mit UV-Strahlen zwecks Polymerisation und Vernetzung, während man die Mischung in einer vorher festgelegten Gestalt hält.

6.  Verfahren wie in Anspruch 5 definiert, in welchem das Vernetzungsmittel mindestens eine Verbindung ist, die aus Divinyl-Verbindungen, Polyepoxy-Verbindungen und Polyaziridinyl-Verbindungen ausgewählt ist.

7.  Phantom für die NMR-Diagnose, dadurch gekennzeichnet, daß das Phantom den in Anspruch 1 definierten Formkörper umfaßt.

8.  Phantom für die NMR-Diagnose wie in Anspruch 7 definiert, welches ein paramagnetisches Metall und/oder eine alkoholische Verbindung enthält.

9.  Verfahren zur Herstellung eines Wasser-absorbierenden Harzes, gekennzeichnet durch Abkühlen und Verfestigen einer Mischung eines Photoinitiators und einer wäßrigen Monomer-Lösung, die ein Alkalimetallsalz von Acrylsäure und ein Vernetzungsmittel enthält, und die Bestrahlung der verfestigten Mischung mit UV-Strahlen zwecks Polymerisation und Vernetzung der Mischung.

10. Verfahren wie in Anspruch 9 definiert, in welchem das Vernetzungsmittel mindestens eine Verbindung ist, die aus Divinyl-Verbindungen, Polyepoxy-Verbindungen und Polyaziridinyl-Verbindungen ausgewählt ist.

**Revendications**

1.  Objet moulé en résine absorbant l'eau, caractérisé en ce que la résine absorbant l'eau est un polymère réticulé d'acide acrylique ou un polymère réticulé d'un sel de métal alcalin d'acide acrylique et forme une phase de matrice uniforme dans laquelle les molécules de la résine sont verrouillées les unes aux autres, et l'eau absorbée par la résine est dispersée uniformément sous forme d'eau liée chimiquement et/ou physiquement à la résine, et qu'il ne peut y avoir plus de trois bulles d'air dont le diamètre ne dépasse pas 0,1 mm, par cm$^3$ de cet objet moulé.

2. Objet moulé suivant la revendication 1, dans lequel la résine absorbant l'eau est un polymère réticulé d'un sel de métal alcalin d'acide acrylique et a une teneur en solubles dans l'eau de 0 à 7% en poids et une capacité d'absorption de 50 à 500 g/g d'eau pure lorsqu'il est sec.

3. Procédé pour la production de l'objet moulé défini dans la revendication 1, caractérisé par le refroidissement, la solidification et le moulage d'un mélange d'un photoinitiateur et d'une solution aqueuse de monomères contenant de l'acide acrylique et/ou un sel de métal alcalin d'acide acrylique et un agent de réticulation, et l'irradiation du mélange moulé avec une radiation ultraviolette pour polymériser et réticuler le mélange.

4. Procédé suivant la revendication 3, dans lequel l'agent de réticulation est au moins un composé choisi parmi des composés divinyles, des composés polyépoxy et des composés polyaziridinyles.

5. Procédé pour la production de l'objet moulé défini dans la revendication 1, caractérisé par l'irradiation d'un mélange d'un photoinitiateur ne libérant pas de gaz dans les conditions de polymérisation et de réticulation, et d'une solution aqueuse de monomères contenant de l'acide acrylique et/ou un sel de métal alcalin d'acide acrylique et un agent de réticulation, avec une radiation ultraviolette pour réaliser la polymérisation et la réticulation afin d'obtenir une résine absorbant l'eau, et le moulage de la résine selon une configuration prédéterminée, ou par l'irradiation du mélange avec une radiation ultraviolette pour réaliser la polymérisation et la réticulation tandis que le mélange est maintenu selon une configuration prédéterminée.

6. Procédé suivant la revendication 5, dans lequel l'agent de réticulation est au moins un composé choisi parmi des composés divinyles, des composés polyépoxy et des composés polyaziridinyles.

7. Fantôme pour le diagnostic par RMN caractérisé en ce que le fantôme comprend l'objet moulé défini dans la revendication 1.

8. Fantôme pour le diagnostic par RMN suivant la revendication 7, qui contient un métal paramagnétique et/ou un composé alcoolique.

9. Procédé pour la production d'une résine absorbant l'eau, caractérisé par le refroidissement et la solidification d'un mélange d'un photoinitiateur et d'une solution aqueuse de monomère contenant un sel de métal alcalin d'acide acrylique et un agent de réticulation, et l'irradiation du mélange moulé avec une radiation ultraviolette pour polymériser et réticuler le mélange.

10. Procédé suivant la revendication 9, dans lequel l'agent de réticulation est au moins un composé choisi parmi des composés divinyles, des composés polyépoxy et des composés polyaziridinyles.